Europäisches Patentamt

European Patent Office

Office européen des brevets

(11) Publication number: **0 215 579**
**A1**

## (12) EUROPEAN PATENT APPLICATION

(21) Application number: **86306340.0**

(22) Date of filing: **15.08.86**

(51) Int. Cl.⁴: **C 07 C 2/00, B 01 J 29/06**

(30) Priority: **21.08.85 GB 8520978**

(43) Date of publication of application: **25.03.87**
**Bulletin 87/13**

(84) Designated Contracting States: **BE DE FR GB IT NL SE**

(71) Applicant: **The British Petroleum Company p.l.c.,**
**Britannic House Moor Lane, London EC2Y 9BU (GB)**

(72) Inventor: **Clayson, David Mark, The British Petroleum**
**Co. p.l.c. Chertsey Road, Sunbury-on-Thames**
**Middlesex, TW16 7LN (GB)**
Inventor: **Ketley, Graham Walter, The British Petroleum**
**Co. p.l.c. Chertsey Road, Sunbury-on-Thames**
**Middlesex, TW16 7LN (GB)**
Inventor: **Hall, Antony Harold Patrick, The British**
**Petroleum Co. p.l.c. Chertsey Road,**
**Sunbury-on-Thames Middlesex, TW16 7LN (GB)**

(74) Representative: **Krishnan, Suryanarayana Kalyana et al,**
**BP INTERNATIONAL LIMITED Patents Division Chertsey**
**Road, Sunbury-on-Thames Middlesex TW16 7LN (GB)**

(54) Production of aromatics from hydrocarbon feedstock.

㊾ This invention relates to a process for producing aromatic hydrocarbons from a mixed feedstock rich in $C_2$ hydrocarbons and containing at least 5 % w/w of methane. The feedstock in the vapour phase and in the absence of oxygen is brought into contact with a gallium loaded zeolite of the MFI type at a temperature from 500–750 °C thereby froming the aromatics. The products formed are useful as gasoline blending components.

EP 0 215 579 A1

1

## PRODUCTION OF AROMATICS FROM HYDROCARBON FEEDSTOCK

The present invention relates to a process for producing aromatic hydrocarbons from a mixed hydrocarbon feedstock rich in $C_2$ hydrocarbons and containing at least 5% by weight of methane.

Hitherto synthetic routes to producing aromatics from open chain hydrocarbons have started from feedstocks which have at least three carbon atoms. Such processes are described for example in our British Patent Nos. 1507778 and 1561590. According to the British Patent 1561590 a gallium catalyst supported on an aluminosilicate in which the ratio of silica to alumina is between 20:1 and 70:1 is used.

More recently our EP-A-0050021 claims and describes a process for converting a feedstock rich in $C_2$ hydrocarbons into aromatics over a gallium loaded zeolite.

It has now been found that the selectivity to aromatics produced from ethane may be improved by the presence of methane in the feed.

Accordingly, the present invention is a process for producing liquid products rich in aromatic hydrocarbons comprising bringing into contact in the vapour phase and in the absence of oxygen at a temperature from 500°C to 750°C a hydrocarbon feedstock containing a major proportion of $C_2$ hydrocarbons and at least 5% by weight of methane with a catalyst composition comprising an aluminosilicate having a silica to alumina molar ratio of at least 5:1 and being loaded with a gallium compound or gallium oxide.

The $C_2$ hydrocarbon in the mixed feedstock may be ethane, ethylene or mixtures thereof. The mixed feed contains at least 5% by weight of methane, preferably from 10-50% by weight of methane. The feedstock may contain in addition other open chain hydrocarbons containing between 3 and 8 carbon atoms as coreactants. Specific examples of such additional coreactants are propane, propylene, n-butane, isobutane, n-butenes and isobutene. The hydrocarbon feedstock (excluding methane) preferably contains at least 70% by weight of $C_2$ hydrocarbons.

The gallium in the catalyst composition may be present as gallium oxide and/or as gallium ions if cations in the aluminosilicate support have been exchanged with gallium ions. Methods of loading aluminosilicates with gallium compounds are described in our EP-A-0024930.

The aluminosilicates which have gallium compounds loaded thereon are suitably zeolites of an MFI type structure (cf. "Chemical Nomenclature, and Formulation of Compositions, of Synthetic and Natural Zeolites," IUPAC yellow booklet, 1978, and zeolite structure types published by The Structure Commission of the International Zeolite Association entitled "Atlas of Zeolite Structure Types", by Meier, W.M. and Olsen, D.H. (1978), distributed by Polycrystal Book Service, Pittsburgh, Pa, USA). The zeolites suitably have a silica to alumina ratio of between 20:1 and 150:1. These may be selected from zeolites of the general formula: $M_{2/n}O.Al_2O_3.ySiO_2zH_2O$ wherein M is a cation which is a positively charged ion selected from a metal ion or an organic ion of valence n and a proton, y is an integer greater than 5 and z is from 0 to 40. The metal cation, M, is preferably an alkali metal or alkaline earth metal ion, preferably sodium or potassium ions. The organic cations may be represented by the formula $R^1R^2R^3R^4N^+$ or by an ion derived from the amine $R^1R^2R^3N$, the diamine $R^1R^2N(CH_2)_xNR^3R^4$ or pyrrolidine where $R^1R^2R^3$ and $R^4$ may be H, $CH_3$, $C_2H_5$, $C_3H_7$, $C_4H_9$ or $-CH_2CH_2OH$ and x equals 2, 3, 4, 5 or 6. A typical example of an MFI type zeolite is ZSM-5. Other zeolites of the ZSM variety, for example ZSM-8, ZSM-11, ZSM-12 and ZSM-35 may also be used. These types of zeolites are extensively described in a number of publications including US

0215579

Patent No. 3970544 (Mobil). These zeolites are usually produced from a silica source, an alumina source, an alkali metal hydroxide and as template a nitrogen containing base. The nitrogen-containing base, may be organic such as an alkanolamine, for example diethanolamine, or inorganic e.g. ammonia. Zeolites made in this manner are described in our published European Patent Application Nos. 0002899, 0002900 and 0030811.

The amount of gallium present in the catalyst compositions may vary for instance between 0.05 and 10% by weight of the total aluminosilicate in the catalyst composition. The gallium loaded zeolite thus obtained may be combined with a porous matrix, e.g. silica or alumina or other inorganic compositions to improve the mechanical strength of the catalyst.

The catalyst composition may be activated prior to contact with the hydrocarbon feedstock. The activation may be carried out by heating the catalyst at a temperature of from 400°C to 750°C, preferably from 500°C to 600°C. Activation may be carried out in an atmosphere of hydrogen, air or a gas inert under the reaction conditions such as nitrogen. The gas may contain or consist of steam. The activation may be carried out in the reactor itself prior to the reaction. The catalyst composition is suitably used in a fixed bed, a moving bed or a fluidised bed.

The hydrocarbon feedstock is thereafter contacted in the vapour phase with the catalyst composition at a temperature from 500° to 750°C preferably from 580° to 650°C in an inert atmosphere in the absence of oxygen. The inert atmosphere may be provided by a gas inert under the reaction conditions such as nitrogen. In fact, once the reactor has been initially flushed with an inert gas such as nitrogen to remove any oxygen or oxidising gases, there is no need to add further amounts of the inert gas to the reaction system. Any unreacted ethane or ethylene recovered from the reaction products may be recycled to the aromatisation reaction.

The reaction is suitably carried out at a pressure from atmospheric to 30 bar, preferably from 3-20 bar.

The WHSV of the feedstock with respect to the catalyst in the reaction is suitably from 0.1-10.

The invention is further illustrated with reference to the following Examples.

In the specification and in the Examples, the term 'selectivity' to aromatics is defined as follows:

$$\frac{\text{weight of aromatics obtained}}{\text{weight of ethane converted}} \times 100$$

Example 1

The catalyst was prepared from a zeolite made according to our published EP-A-0030811. The zeolite was shown to have the MFI structure and to have a silica to alumina mole ratio of 35:1. The zeolite was washed with 10% nitric acid for 30 minutes, filtered, washed with water and dried. It was then refluxed, in two batches, with ammonium nitrate solution, washed and refluxed with gallium nitrate solution. The gallium loaded zeolite was washed with water, dried and bound with an equal part by weight of Ludox 'AS-40' (Registered Trade Mark). The dried cake was broken and sieved to give 12/30 mesh BSS granules which were treated, in a tubular furnace heated to 550°C, with 16.6% vol steam in air at a total GHSV of approximately 285 h$^{-1}$. The resulting catalyst contained 0.79% weight of gallium and contained approximately 30% silica binder.

6g of the gallium loaded, bound catalyst was loaded into a vertical fixed bed reactor. The temperature of the catalyst was raised to 600°C under flowing nitrogen. Hydrogen was then passed over the catalyst for 2 hours before nitrogen was reintroduced. The pressure was then increased to 3 bar absolute and the temperature raised to 625°C. The nitrogen flow was stopped and ethane fed at a rate of 6.9 g/h; giving a contact time of 6 seconds.

A sample of the product stream was analysed by on-line dual column (POROPAK QS and OV101 silicone oil column) gas chromatography, which showed that 39% of the ethane had been converted with a selectivity to aromatics of 56% wt. Selectivity to methane, the major by-product, was 32% wt. Methane was then added to the feed in 18% wt concentration. Simultaneously, the ethane feed rate was reduced sufficiently to maintain the contact time at 6 seconds. On-line analysis showed that 36% of the ethane has been converted with a selectivity to aromatics of 70% wt and a selectivity to methane of 18% w/w.

Claims:

1. A process for producing liquids rich in aromatic hydrocarbons comprising bringing into contact in the vapour phase and in the absence of oxygen at a temperature from 500°C to 750°C a hydrocarbon feedstock containing a major proportion of $C_2$ hydrocarbons and at least 5% w/w of methane with a catalyst composition comprising an aluminosilicate having silica to alumina in a molar ratio of at least 5:1 and being loaded with a gallium compound or gallium oxide.

2. A process according to claim 1 wherein the hydrocarbon feedstock contains at least 50% w/w of ethane, ethylene or mixtures thereof and from 10-50% w/w of methane.

3. A process according to claim 2 wherein the hydrocarbon feedstock comprises a mixture of methane and $C_2$ hydrocarbons.

4. A process according to any one of the preceding claims wherein the aluminosilicate in the catalyst composition is a zeolite having an MFI type structure.

5. A process according to any one of the preceding claims wherein the catalyst composition comprising a gallium loaded aluminosilicate is combined with a porous matrix.

6. A process according to any one of the preceding claims wherein the catalyst composition is activated by heating at a temperature from 400 to 750°C prior to contact with the hydrocarbon feedstock.

7. A process according to any one of the preceding claims wherein the hydrocarbon feedstock is brought into contact with the catalyst composition at a temperature from 580° to 650°C in the vapour phase in the absence of oxygen.

6

8. A process according to any one of the preceding claims wherein the reaction is carried out at a pressure from 1-30 bar.

9. A process according to any one of the preceding claims wherein the WHSV of the reactant hydrocarbon feedstock over the catalyst composition is from 0.1 to 10.

10. A process for producing liquids rich in aromatics according to claim 1 and as herein described with reference to the Examples.

0215579

**PARTIAL EUROPEAN SEARCH REPORT**

which under Rule 45 of the European Patent Convention
shall be considered, for the purposes of subsequent
proceedings, as the European search report

European Patent
Office

Application number

EP 86 30 6340

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| D,X | EP-A-0 050 021 (B.P.)<br>* Claims; pages 2-4 * | 1,4-7 | C 07 C  2/00<br>B 01 J 29/06 |
| X | US-A-4 350 835 (CHESTER et al.)<br>* Claims * | 1,4,5,<br>7 | |
| X | EP-A-0 147 111 (B.P)<br>* Claims; page 2, paragraph 2; page 5, paragraphs 2-4 * | 1,4,8,<br>9 | |

**TECHNICAL FIELDS SEARCHED (Int. Cl.4)**

C 07 C  2/00

## INCOMPLETE SEARCH

The Search Division considers that the present European patent application does not comply with
the provisions of the European Patent Convention to such an extent that it is not possible to carry
out a meaningful search into the state of the art on the basis of some of the claims.

Claims searched completely: 1-9

Claims searched incompletely:

Claims not searched: 10

Reason for the limitation of the search:

See rule 29(6) of the European Patent Conven-
tion.

| Place of search | Date of completion of the search | Examiner |
|---|---|---|
| The Hague | 26-11-1986 | VAN GEYT |

CATEGORY OF CITED DOCUMENTS

X : particularly relevant if taken alone
Y : particularly relevant if combined with another
document of the same category
A : technological background
O : non-written disclosure
P : intermediate document

T : theory or principle underlying the invention
E : earlier patent document, but published on, or
after the filing date
D : document cited in the application
L : document cited for other reasons

& : member of the same patent family, corresponding
document